# EUROPÄISCHE PATENTSCHRIFT

(11) **EP 4 036 567 B1**
(45) Veröffentlichungstag und Bekanntmachung des Hinweises auf die Patenterteilung: **11.03.2026**
(21) Anmeldenummer: 22152384.8
(22) Anmeldetag: 20.01.2022
(51) Int. Cl.: G01N 27/9013

(54) **VERFAHREN ZUR HERSTELLUNG UND PRÜFUNG EINES HOCHFESTEN ROHRPRODUKTES AUS STAHL SOWIE PRÜFSONDE**
METHOD FOR MANUFACTURING AND TESTING A HIGH-STRENGTH STEEL PIPE PRODUCT AND TEST PROBE
PROCÉDÉ DE FABRICATION ET DE VÉRIFICATION D'UN PRODUIT TUBULAIRE HAUTE RÉSISTANCE EN ACIER, AINSI QUE SONDE DE VÉRIFICATION

(30) Priorität: 29.01.2021 DE 102021102086
(43) Veröffentlichungstag der Anmeldung: 03.08.2022
(73) Patentinhaber: Benteler Steel/Tube GmbH & Co. KG, 33104 Paderborn (DE)
(72) Erfinder: Tegethoff, Dirk, 33154 Salzkotten (DE); Thoele, Daniel, 33102 Paderborn (DE); Krause, Harald, 33790 Halle (DE); Luecke, Daniel, 33034 Brakel (DE)
(74) Vertreter: Ksoll, Peter

(56) Entgegenhaltungen:
- DE-A1- 102007 004 223
- DE-A1- 102013 002 775
- US-A- 4 797 613

## Beschreibung

Die Erfindung betrifft ein Verfahren zur Herstellung und Prüfung eines hochfesten Rohrproduktes sowie eine Prüfsonde, insbesondere zur Verwendung in dem Verfahren.

Hochfeste beziehungsweise höchstfeste Rohrprodukte aus Stahl finden in vielen technischen und industriellen Anwendungen Verwendung. Die Herstellung solcher hoch- beziehungsweise höchstfesten Rohrprodukten aus Stahl sind beispielsweise in der EP 3 233 577 B1, der DE 10 2018 106 546 A1, der DE 10 2018 123 316 A1 und der DE 10 2019 103 502 A1 für verschiedene Anwendungen beschrieben. Insbesondere sind dort auch Airbagrohre als solche Rohrprodukte beschrieben.

Derartige hoch- beziehungsweise höchstfeste Rohrprodukte aus Stahl müssen aufgrund ihrer Belastung in den dafür vorgesehenen technischen und/oder industriellen Anwendungen auf ihre Fehlerfreiheit, insbesondere in den während der Herstellung des Rohrproduktes aus einem ursprünglichen Stahlrohr umgeformten Bereichen, geprüft werden. Die Umformung der Rohre kann dabei durch eine Vielzahl gängiger Umformprozesse, wie beispielsweise Warmumformen und Kaltumformen erfolgen.

Im Rahmen dieser Erfindung wird insbesondere die Prüfung umgeformter Endbereiche eines hochfesten Rohrproduktes bearbeitet. Zur Überprüfung stehen dabei verschiedene Möglichkeiten zur Verfügung. Einerseits kann eine Ultraschallprüfung des Rohrproduktes in einer Flüssigkeit (Koppelmedium) durchgeführt werden, wobei hier allerdings zu berücksichtigen ist, dass das geprüfte Rohrprodukt nach der Prüfung zur weiteren Bearbeitung wieder gereinigt und getrocknet werden muss, was einen höheren Energie- beziehungsweise Zeitaufwand bei der Herstellung und Prüfung des Rohrproduktes bedeutet. Zudem können durch die verwendete Flüssigkeit gegebenenfalls Korrosionen an dem hochfesten Rohrprodukt die Folge sein. Außerdem lassen sich durch diese Prüftechnologie nicht alle relevanten Fehlerarten zuverlässig detektieren.

Eine andere Möglichkeit der Prüfung der Oberflächen in den umgeformten Endbereichen eines Rohrproduktes besteht in der Verwendung der Wirbelstromprüfung mit entsprechenden

Rohrproduktes besteht in der Verwendung der Wirbelstromprüfung mit entsprechenden Wirbelstromsensoren. Die Wirbelstromprüfung ist dabei sowohl berührungs- als auch zerstörungsfrei, kann allerdings nur zur Prüfung elektrisch leitender Werkstoffe eingesetzt werden, was aber bei Rohrprodukten aus Stahl der Fall ist. Bei der Wirbelstromprüfung nutzt man den Effekt, dass Verunreinigungen und Beschädigungen in einem elektrisch leitfähigen Material auch eine andere Leitfähigkeit oder eine andere Permeabilität als das eigentliche Material haben. Das Messsignal hängt dabei von den drei Parametern Leitfähigkeit, Permeabilität und Abstand zwischen Detektor und Materialoberfläche ab, sodass damit zum einen Beschädigungen der Oberfläche des zu prüfenden Objektes festgestellt werden können. Zum anderen kann mit der Wirbelstromprüfung auch eine Schichtdickenmessung und eine Prüfung der Materialeigenschaften, insbesondere eine Gefügeprüfung, durchgeführt werden. Im Rahmen der Erfindung wird vorliegend im Wesentlichen auf die Fehlerprüfung der Oberflächen bzw. Wandflächen des hergestellten Rohrproduktes in seinen umgeformten Endbereichen abgestellt.

Bei der Prüfung auf Beschädigung der Oberfläche bzw. Wandfläche wird ein entsprechender Wirbelstromsensor über das zu prüfende Objekt bewegt. Solange dabei der elektrische Widerstand homogen ist und die Wirbelströme daher ungehindert im Material fließen, weist das Material beziehungsweise die Oberfläche keine Beschädigung auf. Sowohl bei Beschädigungen des Materials beziehungsweise der Oberfläche als auch bei einem Einschluss eines Fremdmaterials in der zu überprüfenden Probenwand ändern sich der Widerstand und die Wirbelstromstärke. Diese Änderung kann mit Hilfe der Wirbelstromprüfung detektiert und über eine Auswertelogik mit entsprechenden bildgebenden Verfahren sichtbar oder erkennbar gemacht werden. Die Spulen der verwendeten Wirbelstromsensoren sind dabei so geschaltet, dass kleine Änderungen der Materialeigenschaften oder des Abstands des Wirbelstromsensors von der Materialoberfläche weitgehend kompensiert werden können.

Aus der DE 196 41 888 A1 ist beispielsweise eine Schweißnahtprüfung von Reaktorsteuerstabhülsen in deren Inneren mit einer entsprechenden Wirbelstromsonde offenbart. Weiterhin zeigt die WO 99/04253 A1 eine Innenprüfung einer Rohrinnenwand mittels Wirbelstromprüfung. In der US 10,788,456 B2 ist ebenfalls eine Innenrohrprüfung mittels Wirbelstromprüfung offenbart.

DE 10 2013 002775 A1 offenbart ein Umformwerkzeug für Bauteile, wobei innerhalb des Umformwerkzeugs eine Wirbelstromsonde angeordnet ist, mittels welcher eine Rissprüfung an einem im Umformwerkzeug angeordneten Bauteil durchführbar ist.

Bei all den bekannten Verfahren wird zwar die Oberfläche des Rohrinnenbereichs auf Fehler geprüft. Allerdings können beim Umformen eines Stahlrohres zu einem Rohrprodukt nicht nur an den Innenwänden des Rohrproduktes Beschädigungen auftreten, sondern auch an dessen Außenwänden. Selbst wenn die Innenwände des Rohrproduktes fehlerfrei ausgebildet sind, können Beschädigungen der Oberfläche an der Außenwand des Rohrproduktes, insbesondere in seinen umgeformten Endbereichen zu einer Fehlfunktion bei sachgemäßer Verwendung des Rohrproduktes beziehungsweise auch zur Zerstörung des Rohrproduktes selbst führen. Zwar kann eine derartige Prüfung der Außenwand des Rohrproduktes in seinen umgeformten Endabschnitten ebenfalls erfolgen, jedoch ist dies mit einem erhöhten Zeit-, Material- und Kostenaufwand verbunden.

Es ist daher Aufgabe der Erfindung, ein Verfahren zur Herstellung und Prüfung eines hochfesten beziehungsweise höchstfesten Rohrproduktes aus Stahl zur Verfügung zu stellen, welches eine kostengünstige sowie zeit- und materialsparende Prüfung der umgeformten Endbereiche eines hochfesten beziehungsweise höchstfesten Rohrproduktes aus Stahl ermöglicht. Ferner ist es Aufgabe der Erfindung, eine entsprechende Prüfsonde zur Verwendung in dem erfindungsgemäßen Verfahren und abschließend ein entsprechendes Rohrprodukt zur Verfügung zu stellen.

Gelöst wird diese Aufgabe hinsichtlich des Verfahrens durch ein Verfahren mit allen Merkmalen des Patentanspruchs 1. Bezüglich der Prüfsonde wird die Aufgabe durch eine Prüfsonde mit allen Merkmalen des Patentanspruchs 12 gelöst.

Das erfindungsgemäße Verfahren zur Herstellung und Prüfung eines hochfesten Rohrprodukts aus Stahl weist dabei folgende Verfahrensschritte auf:
a. Bereitstellen eines Stahlrohres, welches sowohl nahtlos als auch mit Schweißnaht ausgeführt sein kann,
b. Umformen des Stahlrohres zum Rohrprodukt, wobei das Stahlrohr wenigstens in einem seiner Endbereiche umgeformt wird,
c. Prüfen der Innenwand und der Außenwand in wenigstens einen umgeformten Endbereich des Rohrproduktes auf Fehler mittels einer an dem wenigstens einen umgeformten Endbereich angepassten Prüfsonde, welche wenigstens einen Wirbelstromsensor zur Prüfung der Innenwand und wenigstens einen Wirbelstromsensor zur Prüfung der Außenwand des wenigstens einen umgeformten Endbereichs des Rohrproduktes aufweist, wobei mehrere Wirbelstromsensoren an wenigstens einem Arm und/oder mehrere Sensoren an einem Innenteil vorgesehen sind.

Durch das erfindungsgemäße Verfahren ist es in einfacher Weise ermöglicht, sowohl die Außenwand als auch die Innenwand eines umgeformten Endbereiches eines Rohrproduktes aus Stahl mittels Wirbelstromprüfung auf Fehler und Beschädigungen zu überprüfen, ohne dass es durch die zusätzliche Überprüfung der Oberfläche im Außenbereich des Endproduktes des Rohrproduktes eines zusätzlichen Zeitbedarfs und auch einen zusätzlichen Prüfstand benötigt. Die Prüfsonde ist derart ausgebildet, dass sie Wirbelstromsensoren aufweist, die gleichzeitig die Oberfläche der Innenwand als auch die Oberfläche der Außenwand des wenigstens einen umgeformten Endbereichs des Rohrproduktes überprüft. Aber nicht nur der Zeitaufwand zur Prüfung der Oberfläche der Außen- und Innenwand ist durch das erfindungsgemäße Verfahren optimiert, vielmehr kann auch bei der zusätzlichen Prüfung der Oberfläche der Außenwand des wenigstens einen umgeformten Endbereichs des Rohrproduktes auf die gleiche Auswerteelektronik zurückgegriffen werden, die auch zur Prüfung der Innenwand des wenigstens einen umgeformten Endbereichs des Rohrproduktes verwendet wird. Dadurch wird erreicht, dass sich insgesamt die Messauflösung und Detektionsrate für bestimmte Fehlerarten verbessert oder gar erst zuverlässig ermöglicht wird, verglichen mit Einzelprüfungen durch Ultraschall oder einseitigen Wirbelstromprüfungen. Schließlich ist dadurch auch noch der apparative Aufwand zur Überprüfung der Innenwand und Außenwand des wenigstens einen umgeformten Endbereichs des Rohrproduktes minimiert. Dabei sind mehrere Sensoren an wenigstens einem Arm und/oder mehrere Sensoren am Innenteil vorgesehen, so dass beispielsweise das Prüfen mit unterschiedlichen Messfrequenzen und/oder Empfindlichkeiten der Wirbelstromsensoren durchführt wird, um unterschiedliche Fehlerarten und/oder Fehler in unterschiedlichen Tiefen der Rohrwand zu ermitteln, oder um zum Beispiel größere Rohrlängenabschnitte gleichzeitig untersuchen zu können.

Im Rahmen der Erfindung ist unter Innenwand die Innenwandfläche bzw. der von der Innenwandoberfläche ausgehende Teil der Rohrwand zu verstehen. Gleichzeitig ist im Rahmen der Erfindung unter Außenwand die Außenwandfläche bzw. der von der Außenwandoberfläche ausgehende Teil der Rohrwand zu verstehen.

Nach einer ersten vorteilhaften Ausgestaltung des erfindungsgemäßen Verfahrens ist es vorgesehen, dass das Prüfen der Innen- und Außenwand gleichzeitig stattfindet. Durch diese parallele Prüfung kann die Prüfungsdauer im Vergleich zu einer seriellen Prüfung mindestens halbiert werden.

Dabei hat sich als besonders vorteilhaft herausgestellt, dass das Prüfen der Innenwand und der Außenwand des wenigstens einen umgeformten Endbereichs des Rohrproduktes derart erfolgt, dass das Rohrprodukt fixiert wird und die Prüfsonde rotierend axial über den wenigstens einen umgeformten Endbereich des Rohrproduktes verfahren wird, wobei mittels der Wirbelstromsensoren sowohl die Innenwand als auch die Außenwand des wenigstens einen umgeformten Endbereichs des Rohrproduktes auf Fehler überprüft wird. Durch die axiale und rotierende Bewegung der Prüfsonde ist sichergestellt, dass die Wirbelstromsonden über die gesamten Oberflächen der Innenwand und der Außenwand des wenigstens einen umgeformten Endbereichs des Rohrproduktes verfahren werden, sodass eine vollständige Prüfung sowohl der Innenwand als auch der Außenwand des wenigstens einen umgeformten Endbereiches gewährleistet werden kann.

Als besonders vorteilhaft hat sich die Ausführung des erfindungsgemäßen Verfahrens bewährt, wonach das gleichzeitige Prüfen der Innenwand und der Außenwand in dem wenigstens einen umgeformten Endbereich des Rohrproduktes direkt im Anschluss an die abschließenden Herstellungsschritte, insbesondere an das Umformen gemäß Schritt b) und insbesondere taktverkettet damit erfolgt. Durch diese Maßnahme kann die Prüfung der Oberflächen der Innenwand und Außenwand des umgeformten Endbereiches im Herstellungsschritt beziehungsweise direkt anschließend daran erfolgen, ohne dass ein separater Prüfstand vorgehalten werden muss. Insofern entfällt auch ein logistischer Aufwand, um die hergestellten Rohrprodukte zusammen einem separaten Prüfstand zuzuführen.

In einer weiteren Ausführung des erfindungsgemäßen Verfahrens ist es vorgesehen, dass das Prüfen im Anschluss an eine Entmagnetisierung des wenigstens einen umgeformten Endbereichs erfolgt. Hierdurch werden Fehldetektionen aufgrund von eventuell im umgeformten Endbereich vorliegenden magnetischen Eigenschaften vermieden.

In einer weiteren vorteilhaften Ausgestaltung des erfindungsgemäßen Verfahrens besteht das bereitgestellte Stahlrohr aus einer Stahllegierung, die außer Eisen und unvermeidbarer schmelzbedingter Verunreinigungen folgende Legierungselemente in Massenprozent aufweist, wobei sich die nachfolgenden Prozentangaben in diesem Dokument sich immer auf Masseprozent beziehen:
C (0,07 bis 0,50%; bevorzugt 0,08 bis 0,15%), Si (0,01 bis 0,60%; bevorzugt 0,01 bis 0,50%), Mn (0,3 bis 1,7%; bevorzugt 1,0 bis 1,7%), Cr (max. 1,2%; bevorzugt 0,2 bis 0,9%), Mo (max. 1,2%; bevorzugt max. 0,2%), Ni (max. 0,4%; bevorzugt 0,15 bis 0,4%), Al (0,01 bis 0,10%), V (max. 0,15%), Nb (max. 0,06%) und Ti (max. 0,06%).

Schmelzbedingte Verunreinigungen sind insbesondere Verunreinigungen, die bei der Stahlherstellung, insbesondere durch beim Erzeugen der Schmelzen und Behandeln der in die Schmelze zugegebene Materialien in die Stahllegierung gelangen
Kohlenstoff (C) liegt erfindungsgemäß in einer Menge im Bereich von 0,07 bis 0,50% in dem Stahl vor. Bei einem Kohlenstoffgehalt von 0,07% kann noch eine ausreichende Festigkeit gewährleistet werden und die Zementitbildung Fe₃C im Stahl geringgehalten werden. Zudem kann eine ausreichende Zähigkeit sichergestellt werden. Bei einem zu hohen Kohlenstoffgehalt wird hingegen die Karbidbildung in dem Stahl begünstigt, wodurch die Kerbschlagzähigkeit sinkt. Der Kohlenstoffgehalt ist daher erfindungsgemäß vorzugsweise auf maximal 0,15% beschränkt. Gemäß einer Ausführungsform kann der Kohlenstoffgehalt in einem Bereich von 0,08 bis 0,15 % liegen.

Silizium (Si) liegt vorzugsweise in einer Menge im Bereich von 0,01 bis 0,60%, vorzugsweise im Bereich von 0,01 bis 0,50%, vor. Silizium erhöht die Zugfestigkeit und Streckgrenze des bereitgestellten Stahlrohrs.

Mangan (Mn) liegt vorzugsweise in einer Menge im Bereich von 0,3 bis 1,7% vor. Mangan erhöht die Streckgrenze und Festigkeit der Stahllegierung. Zudem verbessert Mangan als Ersatz für Kohlenstoff die Schweißbarkeit. Gemäß einer bevorzugten Ausführungsform liegt Mangan in einer Menge im Bereich von 0,5 bis 1,7% und besonders bevorzugt im Bereich von 0,6 bis 1,7% vor.

Chrom (Cr) liegt vorzugsweise in einer Menge von maximal 1,2% vor. Chrom erhöht die Zähigkeit und Zugfestigkeit der Stahllegierung. Gemäß einer bevorzugten Ausführungsform liegt Chrom in einer Menge im Bereich von 1,0% und besonders bevorzugt im Bereich von 0,2 bis 0,9% vor.

Molybdän (Mo) liegt vorzugsweise in einer Menge von maximal 1,2%, insbesondere maximal 0,2% vor. Molybdän verbessert insbesondere die Zugfestigkeit und Schweißbarkeit der Stahllegierung.

Nickel (Ni) liegt vorzugsweise in einer Menge von maximal 0,4%, bevorzugt zwischen 0,15 und 0,4% vor. Nickel erhöht die Zugfestigkeit und die Streckgrenze.

Aluminium (Al) liegt vorzugsweise in einer Menge im Bereich von 0,01 0,10% vor.

Vanadium (V) liegt vorzugsweise in einer Menge von maximal 0,15% vor. Vanadium erhöht die Zugfestigkeit der Legierung.

Niob (Nb) liegt vorzugsweise in einer Menge von maximal 0,06% vor.

Titan (Ti) liegt vorzugsweise in einer Menge von maximal 0,06% vor.

In einer anderen Ausgestaltung des erfindungsgemäßen Verfahrens weist das Rohrprodukt eine Gefügestruktur aus gestrecktem, angelassenem Martensit auf, insbesondere mit einer durchschnittlichen martensitischen Paketgröße von d_{avg} < 3µm. Derartige Eigenschaften des Rohproduktes können beispielsweise durch ein Vergüten, d.h. durch Härten und anschließendem Anlassen) und einem Kaltziehen nach dem Vergüten, insbesondere vor dem in Schritt b) beschriebenen Prüfen realisiert werden.

Gemäß einer weiteren Ausführungsform des erfindungsgemäßen Verfahrens weist das Rohrprodukt, insbesondere das Airbagrohr eine Gefügestruktur aus gestrecktem, angelassenem Martensit auf. Diese Gefügestruktur wird vorzugsweise insbesondere durch eine Wärmebehandlung mit anschließendem Kaltziehen erzielt.

Gemäß einer alternativen Ausführungsform kann ein Rohrprodukt, insbesondere ein Airbagrohr auch aus einer lufthärtbaren Stahllegierung bestehen, wie sie beispielsweise in der EP 1 474 538 A1 offenbart ist. Diese Stahllegierung sowie die darin offenbarten Rohrherstellungsschritte bilden dabei einen Teil der vorliegenden Offenbarung hinsichtlich des Schritts a) des Patentanspruchs 1, nämlich der Bereitstellung des Stahlrohrs.

Bevorzugt weist das Rohrprodukt eine Übergangstemperatur von kleiner 233,15 K auf. Die Übergangstemperatur wird vorzugsweise durch den Ring-Charpy-Versuch ermittelt. Beispielsweise wird hierbei die Übergangstemperatur ermittelt durch eine Probennahme in jeweiligen Längenabschnitt als ringförmige Kerbschlagprobe, das heißt, als schmaler Rohrabschnitt mit einer zu Testzwecken eingebrachten Sollkerbe. Nach Abkühlung auf Tieftemperaturbedingung des Tests erfolgt ein axialer Schlag auf die Probe. Bei derjenigen Tieftemperatur, bei der von einem plastischen auf ein sprödes Bruchverhalten der Proben übergegangen wird, spricht man von der Übergangstemperatur. Es versteht sich, dass zu deren Ermittlung mehrere Proben für die Charakterisierung eines Längenabschnitts getestet werden müssen.

Neben Airbagrohren können folgende weitere Rohranwendungen mit dem erfindungsgemäßen Verfahren hergestellt beziehungsweise in diesem verwendet werden. Antriebswellen, Stabilisatoren und Achskomponenten insbesondere aus einer lufthärtenden Stahllegierung, wie sie in der DE 10 2017 297 369 A1, DE 10 2016 107 143 A1 sowie DE 10 2015 111 150 A1 offenbart werden. Die darin offenbarten Stahllegierungen sowie Rohrherstellungsschritte bilden dabei einen Teil der vorliegenden Offenbarung hinsichtlich des Schritts a) des Patentanspruchs 1, nämlich der Bereitstellung des Stahlrohrs, sowie den Schritt b), der Umformung des Stahlrohrs zu Rohrprodukt.

Weiterhin bevorzugt weist das Rohrprodukt und/oder das bereitgestellte Stahlrohr eine Wandstärke von weniger als 4 mm auf. Eine derartige Wandstärke kann insbesondere auch bei Airbagrohren zum Einsatz kommen.

Besonders vorteilhaft ist es, wenn ein Prüfen auf Fehler im Übergangsbereich durch Verfahren eines Wirbelstromsensors im Hinterschnitt des Übergangsbereichs der Innen- oder Außenwand erfolgt. Hierdurch ist gewährleistet, dass die Wirbelstromsensoren im Übergangsbereich an diesen herangeführt werden können, sodass sie sich dort in einem Abstand zur Wand des Übergangsbereichs befinden, die eine Detektion und somit ein Prüfen ermöglichen. Dieses Heranführen kann durch entsprechende Mechaniken in den Armen und/oder dem Innenteil realisiert werden. Nur beispielsweise sei hierbei ein Spreizen eines Sensorhalters, ein querbewegter Sensorhalter oder auch eine segmentierte Prüfsonde genannt, wobei diese Auflistung nicht abschließend ist.

Grundsätzlich kann im Rahmen des erfindungsgemäßen Verfahrens auch eine Abstandskompensation integriert sein. Hierdurch wird, beispielsweise in der Fehlerauswertung, der Elektronik und/oder der verwendeten Software, eine rotations- und/oder einspannbedingte Abstandsvariation zwischen den Wirbelstromsensoren und den Wandoberflächen kompensiert.

Die erfindungsgemäße Prüfsonde zeichnet sich dadurch aus, dass sie einen Steg aufweist, der wenigstens einen Arm, an dessen Innenwand wenigstens ein Wirbelstromsensor angeordnet ist, und ein Halteelement zur Aufnahme wenigstens eines Wirbelstromsensors miteinander verbindet, wobei die Prüfsonde als Rotationskörper um eine Mittellängsachse ausgebildet, wobei der Stegbereich als Kreisscheibe, die beiden Arme zusammen als auf dem Rand der Kreisscheibe angeordneter Hohlzylinder und das Halteelement als Vollzylinder um die Mitte der Kreisscheibe auf dieser angeordnet ausgebildet sind. Durch eine derartige aufgebaute Prüfsonde ist in einfacher Weise ein gleichzeitiges Prüfen der Außen- und Innenwand eines rohrförmigen Elementes, insbesondere eines rohrförmigen Produkts, hergestellt nach dem vorhergehend beschriebenen Verfahren, gegeben. Durch die auf den Innenwänden des ersten beziehungsweise zweiten Arms, welche auf dem Steg angeordnet sind, angeordneten Wirbelstromsensoren kann in einfacher Weise die Oberfläche der Außenwand eines Endbereichs eines Rohrproduktes geprüft werden, während der wenigstens eine auf dem Halteelement angeordnete Wirbelstromsensor zur Überprüfung der Innenwand des Endbereichs eines Rohrproduktes ausgebildet ist. Durch Rotation und axialem Verfahren der Prüfsonde über den Endbereich des Rohrproduktes kann somit die gesamte Oberfläche der Innenwand und der Außenwand mittels der Prüfsonde auf Fehler und Beschädigungen überprüft werden. Eine derartige Prüfsonde kann in einfacher Weise aus einem massiven Metallkörper geformt werden, insbesondere gefräst werden, wobei es sich als besonders vorteilhaft erweist, dass ein derartiger Vollkörper ein hohes Gewicht hat und daher relativ unempfindlich gegen auftretende leichte Unwuchten bei der Rotation ist. Dies ist insbesondere deshalb wichtig, da die einzelnen Wirbelstromsensoren der Prüfsonde an unterschiedlichen Orten des Rotationskörpers angeordnet sind und daher bei einer Rotation leichte Unwuchten auftreten könnten. Bei einer massiven Prüfsonde, insbesondere aus einem Metall fallen daher die Gewichte der Wirbelstromsensoren, sodass die durch sie erzeugten leichten Unwuchten bei der Rotation der als Rotationssonde ausgebildeten Prüfsonde nicht störend sind. Eine derartig als Rotationskörper ausgebildete Prüfsonde wird nachfolgend gegebenenfalls auch als Topfsonde bezeichnet.

Von besonderem Vorteil ist dabei, wenn das Halteelement äquidistant zu dem ersten und dem zweiten Arm auf dem Steg angebracht ist. Durch diese Ausgestaltung der Erfindung ist sichergestellt, beim Rotieren der Arme über dem Endbereich des Rohrproduktes beide Arme den gleichen Abstand zu der Außenwand des Rohrproduktes aufweisen. Insofern würden dann auch auf den beiden Armen direkt gegenüberliegende Wirbelstromsensoren exakt gleiche Signale liefern, sodass diese miteinander vergleichbar wären und auch eine redundante Prüfung möglich wäre. Allerdings ist eine gegenüberliegende Anordnung von Wirbelstromsensoren auf den beiden Armen nicht vorgesehen, da ein Defekt eines Wirbelstromsensors auch anderweitig feststellbar wäre beziehungsweise sofort auffallen würde. Dabei kann die Geometrie der Innenwände der beiden Arme an die Geometrie des zu überprüfenden Endbereichs eines Rohrprodukts angepasst werden, sodass durch entsprechend angeordnete Wirbelstromsensoren diese immer den gleichen Abstand zur überprüfenden Oberfläche des umgeformten Endbereichs des Rohrprodukts aufweisen.

Alternativ ist es möglich, dass die Prüfsonde gerade nicht die Geometrie eines Rotationskörpers aufweist. Insbesondere dann, wenn sie nur einen Arm zur Prüfung einer Außenwand aufweist. Das Halteelement zur Prüfung von Innenwänden kann ebenfalls als Arm ausgebildet sein, der von dem Steg der Prüfsonde absteht und in das Innere eines zu prüfenden Rohres eingeführt werden kann.

Dabei kann die Prüfsonde senkrecht zur Rotationsachse des zu prüfenden Rohres verfahren werden, um den Arm zur Prüfung von Außenwänden und das Halteelement zur Prüfung von Innenwänden an Hinterschnitte an den Enden des zu prüfenden Rohres herangefahren werden. Die Geometrie des Armes und des Halteelementes ist dabei an die Geometrie des zu prüfenden Rohrendes angepasst. Wenn nunmehr die Prüfung des Rohrendes stattfindet entspricht die Rotationsachse der Prüfsonde der Mittellängsachse des zu prüfendes Rohres.

Weitere Ziele, Vorteile, Merkmale und Anwendungsmöglichkeiten der vorliegenden Erfindung ergeben sich aus der nachfolgenden Beschreibung von Ausführungsbeispielen anhand der Zeichnungen. Dabei bilden alle beschriebenen und/oder bildlich dargestellten Merkmale für sich oder in beliebiger sinnvoller Kombination den Gegenstand der vorliegenden Erfindung, auch unabhängig von ihrer Zusammenfassung in den Ansprüchen oder deren Rückbeziehung.

Es zeigen:
- Figur 1:: Darstellung zu Beginn des Prüfschritts des erfindungsgemäßen Verfahrens zur Prüfung zweier umgeformter Endbereiche eines erfindungsgemäßen Rohrproduktes mit erfindungsgemäßen Prüfsonden,
- Figur 2:: Darstellung der Prüfung der Endabschnitte des Rohrprodukts der Figur 1 zum Ende der Prüfung
- Figur 3: Darstellung der Prüfung eines Endabschnitte eines weiteren Rohrprodukts zum Ende der Prüfung mit einer weiteren erfindungsgemäßen Prüfsonde,
- Figur 4: Darstellung der Prüfung eines Endabschnitte eines weiteren Rohrprodukts zum Ende der Prüfung mit einer weiteren erfindungsgemäßen Prüfsonde,
- Figur 5: Darstellung der Prüfung eines Endabschnitte eines weiteren Rohrprodukts zum Ende der Prüfung mit einer weiteren erfindungsgemäßen Prüfsonde und
- Figur 6:: Darstellung verschiedener Fehlerarten, die in einen erfindungsgemäßen Rohrprodukt auftreten können.

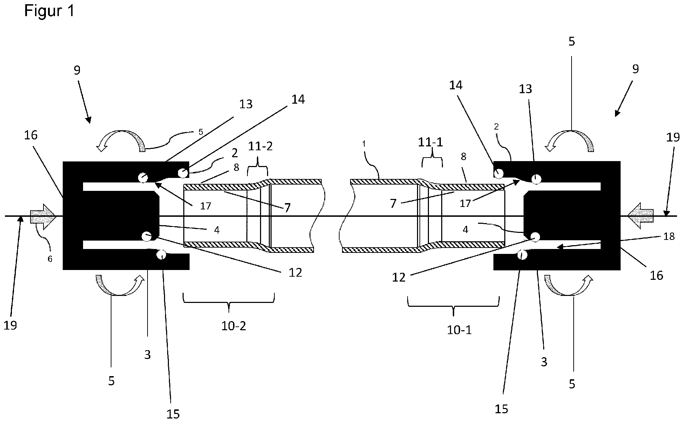

In Figur 1 wird ein erfindungsgemäßes Rohrprodukt 1, insbesondere ein Airbagrohr, mit umgeformten Endbereichen 10-1, 10-2 dargestellt, welche mittels jeweils einer Prüfsonde 9 auf Fehler der Innenwand 7 und der Außenwand 8 in den umgeformten Endbereichen 10-1, 10-2 des Rohrproduktes 1 geprüft werden sollen. Die Endbereiche weisen einen Übergangsbereich 11-1, 11-2 zwischen dem umgeformten beziehungsweise reduzierten Querschnitt des Rohrproduktes 1 und dem Querschnitt des bereitgestellten Stahlrohrs auf. Die Darstellung der Figur 1 zeigt den Beginn der Prüfung. Das Rohrprodukt 1 ist dabei derart ausgebildet, dass während seiner Herstellung seine Endbereiche 10-1, 10-2 verjüngt wurden. In weiteren hier nicht dargestellten Ausführungsbeispielen ist es auch denkbar, dass die Endbereiche des Rohrproduktes aufgeweitet wurden. Die Umformung bei der Herstellung des Rohrproduktes aus einem Stahlrohr kann dabei nach bekannten hier nicht näher erläuterten Verfahren erfolgen.

Die Endbereiche 10-1 und 10-2 des Rohrproduktes 1 sind dabei rotationssymmetrisch mit einer Innenwand 7 und einer Außenwand 8 ausgebildet. Zur Prüfung auf Fehler an den Innenwänden 7 und Außenwänden 8 in den Endbereichen 10-1 und 10-2 des Rohrproduktes 1 werden vorliegend zwei Prüfsonden 9 verwendet. Die Prüfsonden 9 bestehen dabei aus einem Steg 16, welcher einen ersten Arm 2 und einen zweiten Arm 3 miteinander verbindet, wobei auf dem Steg 16 ein Halteelement 4 angeordnet ist, welches äquidistant von den beiden Armen 2 und 3 beabstandet ist. Auf den beiden Armen 2 und 3 sind Wirbelstromsensoren 13, 14 und 15 angeordnet, die derart positioniert sind, dass sie Bereiche verschiedenen Durchmessers der Endbereiche 10-1, 10-2 des Rohrproduktes 1 prüfen können, wobei die Abstände zwischen Wirbelstromsensor 13, 14 und 15 sowie dem jeweiligen Bereich der Außenwände 8 im Wesentlichen die gleichen Abstände aufweisen. Auf dem Halteelement 4 ist ebenfalls ein Wirbelstromsensor 12 angeordnet, mit dem die Innenwände 7 der Endbereiche 10-1 und 10-2 des Rohrproduktes auf Fehler überprüft werden.

Da die Darstellung der Figur 1 in Art einer Schnittdarstellung gezeigt ist, sei zu den Prüfsonden 9 noch angemerkt, dass es sich hierbei um massive Elemente aus Metall in Form eines Rotationskörpers handelt, sodass der Steg 16 als Kreisscheibe ausgebildet ist, während die beiden Arme 2 und 3 zusammen einen Hohlzylinder bilden und auf dem als Kreisscheibe ausgebildeten Steg 16 an dessen Rand angeordnet sind und. In gleicher Weise ist das Halteelement 4 jeweils als Vollzylinder auf dem als Kreisscheibe ausgebildeten Steg16 ausgebildet und auf dessen Mitte angeordnet. Zwar sind die Endbereiche 10-1, 10-2 gemäß Figur 1 nach dem Umformen identisch und weisen den gleichen Querschnitt und gleiche Übergangsbereiche 11-1, 11-2 auf, die Querschnittsgeometrie, die Länge sowie die Breite der Übergangsbereiche 11-1 und 11-2 können jedoch auch unterschiedlich sein. Dementsprechend können auch die Prüfsonden 9 trotz gleicher Bezugszeichen im Detail unterschiedlich, insbesondere an die Geometrie der Endbereiche 10-1, 10-2 angepasst ausgebildet, sein. Zudem müssen erfindungsgemäße Prüfsonden 9 auch nicht als Rotationskörper ausgebildet sein. Wenn sie als Rotationskörper ausgebildet sind, müssen sie allerdings nicht unbedingt aus massiven Elementen aus Metall ausgebildet sein. Denkbar wären beispielsweise auch Hohlelemente mit Blechwänden aus Metall. Zudem ist das Material, aus dem die Prüfsonde besteht, auch nicht auf Metalle beschränkt.

Wie bereits erwähnt, zeigt die Darstellung der Figur 1 die Situation zu Beginn der Prüfung der Endbereiche 10-1 und 10-2 des Rohrproduktes 1 auf Fehler. Die Prüfsonden 9 befinden sich derweil bereits in Rotation, wobei die Pfeile 5 die Rotationsrichtung angeben und die Rotation um die Mittellängsachse 19 erfolgt. Zusätzlich zur Rotationsbewegung 5 erfahren die Prüfsonden 9 auch noch eine axiale Bewegung, die durch die Pfeile 6 angedeutet ist.

Während der axialen Bewegung der Prüfsonden 9 rotieren diese um die Mittellängsachse 19 weiter, sodass mittels der Wirbelstromsensoren 12 bis 15 die Oberflächen der Außenwände 8 und Innenwände 7 in den Endbereichen 10-1 und 10-2 des Rohrproduktes 1 über ihre gesamte Fläche auf Fehler und Beschädigungen überprüft werden können.

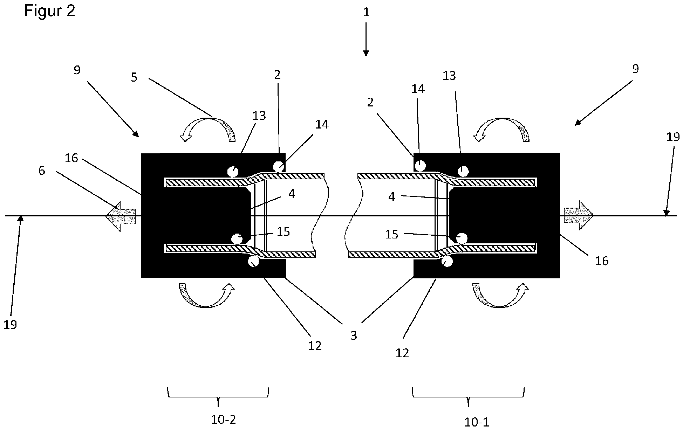

Das Verfahren der Prüfsonden 9 in axialer Richtung erfolgt dabei so lange, bis die Prüfsonden 9 die in Figur 2 dargestellte Position erreicht haben. In dieser Position ist das Rohrprodukt 1 mit seinen Endbereichen 10-1, 10-2 vollständig in die Prüfsonden 9 eingetaucht, sodass die Außen- und Innenwände 8 und 7 des Rohrproduktes 1 bereits einmal vollständig überprüft wurden. Die Überprüfung ist hiermit abgeschlossen und die Prüfsonden 9 können wieder in ihre Ausgangsposition gemäß Figur 1 verfahren werden. Dabei kann nunmehr bereits auf eine Rotation der Prüfsonden 9 verzichtet werden, da bereits eine vollständige Überprüfung der Oberflächen der Innenwände 7 und der Außenwände 8 in den Endbereichen 10-1 und 10-2 des Rohrproduktes erfolgt ist. Alternativ ist es möglich, auch während des Herausfahrens der Prüfsonden 9 aus den Endbereichen 10-1 und 10-2 des Rohrproduktes 1 eine nochmalige Überprüfung der Oberflächen an den Innenwänden 7 und den Außenwänden 8 des Rohrproduktes 1 durchzuführen.

Sofern während der Überprüfung der Endbereiche 10-1 und 10-2 des Rohrproduktes 1 keine Fehler festgestellt wurden, wird das Rohrprodukt 1 seiner weiteren Verwendung zugeführt. Sollten jedoch Fehler oder Beschädigungen bei der Überprüfung detektiert worden sein, wird das entsprechende Rohrprodukt 1 aussortiert.

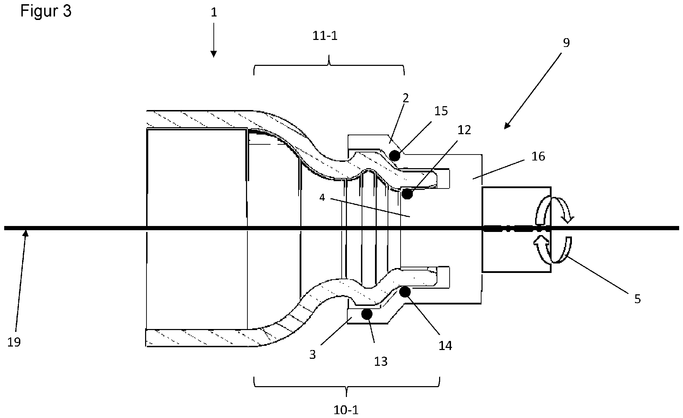

In der Figur 3 ist eine Prüfung eines Endabschnitte eines weiteren Rohrprodukts 1 zum Ende der Prüfung mit einer weiteren erfindungsgemäßen Prüfsonde 9 dargestellt. Die Prüfsonde 9 entspricht in ihrem Aufbau im Wesentlichen der der Figuren 1 und 2 und ist daher mit identischen Bezugszeichen versehen. Das Rohrprodukt 1 der Figur 3 ist in ihrem Endbereich 10-1 allerdings unterschiedlich zum dem Endbereich des Rohrproduktes der Figuren 1 und 2 und ist bin einem Übergangsbereich 11-1 zwar auch verjüngend ausgebildet, weist aber zudem eine nach außen ausgebildete Wulst auf. Die Prüfung erfolgt analog zu der in den Figuren 1 und 2 beschriebenen Prüfung, sodass hier auf eine erneute Ausführung verzichtet wird.

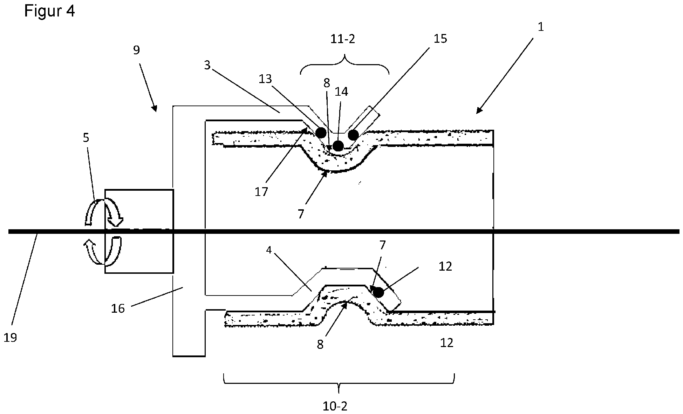

Die Figur 4 zeigt eine Prüfung eines Endabschnitte eines weiteren Rohrprodukts 1 zum Ende der Prüfung mit einer weiteren erfindungsgemäßen Prüfsonde 9. Das dortige Rohrprodukt ist in seinem Endbereiche 10-2 diesmal nicht verjüngend ausgebildet, sondern weist in einem Übergangsbereich 11-2 eine zum Rohrinneren hingerichtet Wulst auf. Die hier verwendete Prüfsonde 9 ist diesmal nicht um ihre Rotationsachse, die der Mittellangsachse 19 des Rohrproduktes entspricht, rotationssymmetrisch ausgebildet. Vielmehr weist die Prüfsonde 9 einen Steg 16 auf, an dessen einem Ende ein Arm 3 angeordnet ist, der an seiner Innenwand 17 mit drei Wirbelstromsensoren 13, 14, und 15 versehen ist, um die Außenwand 8 des Rohrproduktes im Bereich der Wulst zu Prüfen. Ferner weist die Prüfsonde eine Halteelement 4 auf, das hier ebenfalls als Arm ausgebildet und auf dem Steg 16 gegenüber dem Arm 3 angeordnet ist. Diese Halteelement 4 ist mit einem Wirbelstromsensor 12 zur Prüfung der Innenwand 7 des Rohrproduktes 1 im Bereich der Wulst des Endbereichs 10-2 bestückt. Der Arm 3 und das Halteelement 4 sind dabei unterschiedlich weit von der Rotationsachse der Prüfsonde 9 beabstandet sodass bei Rotation der Prüfsonde 9 das Halteelement 4 an der Außenwand 8 des Rohrproduktes 1 in dessen Endbereich 10-2 entlanggeführt wird, während das Haltelement an der Innenwand 7 des Rohrproduktes 1 in dessen Endbereich 10-2 entlanggeführt wird.

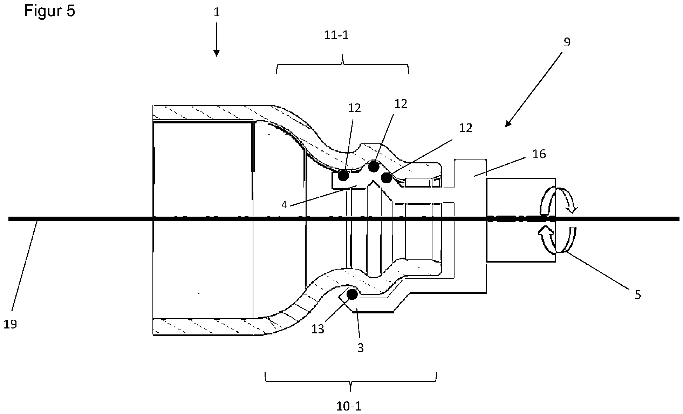

Die Figur 5 zeigt eine Prüfung eines Endabschnitte eines weiteren Rohrprodukts 1 zum Ende der Prüfung mit einer weiteren erfindungsgemäßen Prüfsonde 9. Das dortige Rohrprodukt ist in seinem Endbereiche 10-1 diesmal nicht verjüngend ausgebildet, sondern weist in einem Übergangsbereich 11-1 eine zum Rohräußeren hingerichtet Wulst auf. Die hier verwendete Prüfsonde 9 ist diesmal nicht um ihre Rotationsachse, die der Mittellangsachse 19 des Rohrproduktes entspricht, rotationssymmetrisch ausgebildet. Vielmehr weist die Prüfsonde 9 einen Steg 16 auf, an dessen einem Ende ein Arm 3 angeordnet ist, der an seiner Innenwand 17 mit einem Wirbelstromsensor 13 versehen ist, um die Außenwand 8 des Rohrproduktes im Bereich der Wulst zu Prüfen. Ferner weist die Prüfsonde eine Halteelement 4 auf, das hier ebenfalls als Arm ausgebildet und auf dem Steg 16 gegenüber dem Arm 3 angeordnet ist. Diese Halteelement 4 ist mit drei Wirbelstromsensor 12, 12 und 12 zur Prüfung der Innenwand 7 des Rohrproduktes 1 im Bereich der Wulst des Endbereichs 10-1 bestückt. Der Arm 3 und das Halteelement 4 sind dabei unterschiedlich weit von der Rotationsachse der Prüfsonde 9 beabstandet sodass bei Rotation der Prüfsonde 9 das Halteelement 4 an der Außenwand 8 des Rohrproduktes 1 in dessen Endbereich 10-1 entlanggeführt wird, während das Haltelement an der Innenwand 7 des Rohrproduktes 1 in dessen Endbereich 10-1 entlanggeführt wird.

Die in Figur 4 und 5 beschriebenen Ausführungsformen der Erfindung werden wie folgt geprüft. Vor und/oder während der Prüfung des Rohrprodukt 1 wird die Prüfsonde 9 derart in und über das zu prüfende Endbereich 10-1 bzw. 10-2 bewegt, dass zeitgleich oder nacheinander entlang einer Mittellangsachse 19 des Rohrprodukts 1 und radial zur der Rohrachse 19 geführt wird, um den durch den Übergangsbereich 11-1 bzw. 11-2 ausgebildeten Hinterschnitt mit dem Arm 3 und Halteelement 4 kollisionsfrei zu erreichen. Nach Messungsende wird entsprechend die Prüfsonde 9 vom Endbereich 10-2 parallel und quer zur Mittellangsachse 19 kollisionsfrei weggeführt.

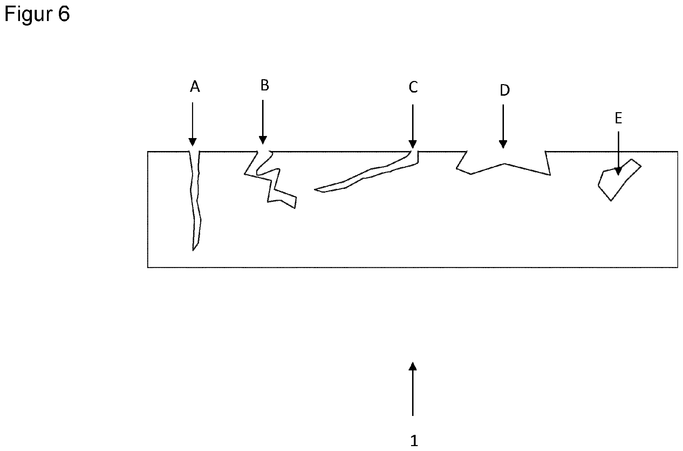

In der Figur 6 sind beispielhaft Fehler in einer Wand eines erfindungsgemäßen Rohrproduktes 1 beispielhaft und nicht abschließend dargestellt, die mittels einer Prüfung mit dem erfindungsgemäßen Verfahren detektiert werden können. Hierbei ist möglich, sowohl verschiedene Oberflächenfehler A, B, C, D als auch Einschlussfehler E bei einer Prüfung sicher zu detektieren und mit bekannten Verfahren auf bekannten bildgebenden Vorrichtungen wie beispielsweise Bildschirmen zu visualisieren.

Hinsichtlich der Einschlussfehler E hängt die Detektionsrate auch von der Leistung der Wirbelstromsensoren ab, mit welchen das Rohrprodukt 1 geprüft wird. Dabei gilt grundsätzlich, je höher die Leistung, desto größer auch die Detektionstiefe.

Bei dem Oberflächenfehler A handelt es sich um einen mehr oder weniger senkrechten Riss in de Oberfläche des Rohrproduktes 1, während der Oberflächenfehler B einen Riss darstellt, der mehrfach seine Richtung ändert, je größer die Eindringtiefe ist. Der Oberflächenfehler C ist ein mehr oder weniger gerade verlaufender Riss, der im Gegensatz zu Oberflächenfehler A allerdings nicht rechtwinklig zur Oberfläche des Rohrproduktes verläuft. Bei dem Oberflächenfehler D handelt es sich weniger um einen Riss in der Oberfläche des Rohrproduktes, da seine Eindringtiefe im Gegensatz zu den Oberflächenfehlern A, B, C relativ gering ist. Vielmahre ist der Oberflächenfehler D in Form eines kraterförmigen Oberflächenfehlers ausgebildet.

Alle in Figur 6 gezeigten Oberflächenfehler lassen sich mit dem erfindungsgemäßen Verfahren sicher und präzise detektieren. Im Vergleich zur Ultraschallprüfung hat die Wirbelstromprüfung den Vorteil, dass sie trocken durchgeführt werden kann. Insofern ergibt sich gegenüber der Ultraschallprüfung nicht nur ein Zeiteinsparung durch die der Wirbelstromprüfung innewohnenden schnellen Oberflächenprüfung im Bereich von wenigen Sekunde. Vielmehr entfällt auch der Zeitaufwand zur Trocknung des Rohrproduktes, wobei bei der Ultraschallprüfung durch das nasse Prüfverfahren auch Korrosionsprobleme bei den Rohrprodukten auftreten können, die bei eine Wirbelstromprüfung nicht auftreten können.

### Bezugszeichenliste

- 1: Rohrprodukt
- 2: erster Arm
- 3: zweiter Arm
- 4: Halteelement
- 5: Rotationsbewegung
- 6: axiale Bewegung
- 7: Innenwand
- 8: Außenwand
- 9: Prüfsonde
- 10-1: Endbereich
- 10-2: Endbereich
- 11-1: Übergangsbereich
- 11-2: Übergangsbereich
- 12: Wirbelstromsensor
- 13: Wirbelstromsensor
- 14: Wirbelstromsensor
- 15: Wirbelstromsensor
- 16: Steg
- 17: Innenwand
- 18: Innenwand
- 19: Mittellängsachse
- A: Oberflächenfehler
- B: Oberflächenfehler
- C: Oberflächenfehler
- D: Oberflächenfehler
- E: Einschlussfehler

## Patentansprüche

1. Verfahren zur Herstellung und Prüfung eines Rohrproduktes (1) aus Stahl mit folgenden Schritten:
a) Bereitstellen eines Stahlrohres,
b) Umformen des Stahlrohres zum Rohrprodukt (1), wobei das Stahlrohr wenigstens in einem seiner Endbereiche (10-1, 10-2) umgeformt wird,
c) Prüfen der Innenwand (7) und der Außenwand (8) in wenigstens einem umgeformten Endbereich (10-1, 10-2) des Rohrproduktes (1) auf Fehler mittels wenigstens einer an wenigstens einem umgeformten Endbereich (10-1, 10-2) angepassten Prüfsonde (9), welche wenigstens einen Wirbelstromsensor (12) zur Prüfung der Innenwand (7) und wenigstens einen Wirbelstromsensor (13, 14, 15) zur Prüfung der Außenwand (8) des wenigstens einen umgeformten Endbereichs (10-1, 10-2) des Rohrproduktes aufweist, wobei mehrere Wirbelstromsensoren (13, 14, 15) an wenigstens einem Arm (2, 3) und/oder mehrere Sensoren (12) an einem Innenteil (4) vorgesehen sind.

2. Verfahren nach Anspruch 1, **dadurch gekennzeichnet, dass** im Schritt c) gleichzeitig die Innenwand und die Außenwand geprüft wird.

3. Verfahren nach Anspruch 1 oder 2, **dadurch gekennzeichnet, dass** das Prüfen der Innenwand (7) und der Außenwand (8) des wenigstens einen umgeformten Endbereichs (10-1, 10-2) des Rohrproduktes (1) derart erfolgt, dass das Rohrprodukt (1) fixiert wird und die Prüfsonde (9) rotierend axial über den wenigstens einen umgeformten Endbereich (10-1, 10-2) des Rohprodukts verfahren wird, wobei mittels der Wirbelstromsensoren (12, 13, 14, 15) sowohl die Innenwand (7) als auch die Außenwand (8) des wenigstens einen umgeformten Endbereich (10-1, 10-2) des Rohrproduktes auf Fehler geprüft wird.

4. Verfahren nach einem der vorhergehenden Ansprüche, **dadurch gekennzeichnet, dass** das Prüfen der Innenwand (7) und der Außenwand (8) in dem wenigstens einen umgeformten Endbereich (10-1, 10-2) des Rohrproduktes (1) im Anschluss an die abschließenden Herstellungsschritte, insbesondere an das Umformen gemäß Schritt b) und insbesondere taktverkettet damit erfolgt.

5. Verfahren nach einem der vorhergehenden Ansprüche, **dadurch gekennzeichnet, dass** das Prüfen im Anschluss an eine Entmagnetisierung des wenigstens einen umgeformten Endbereichs erfolgt.

6. Verfahren nach einem der vorhergehenden Ansprüche, **dadurch gekennzeichnet, dass** das bereitgestellte Stahlrohr aus einer Stahllegierung besteht, die außer Eisen und schmelzbedingter Verunreinigungen folgende Legierungselemente in Massenprozent aufweist:
C (0,07 bis 0,50%; bevorzugt 0,08 bis 0,15%),
Si (0,01 bis 0,60%; bevorzugt 0,01 bis 0,50%),
Mn (0,3 bis 1,7%; bevorzugt 1,0 bis 1,7%),
Cr (max. 1,2%; bevorzugt 0,2 bis 0,9%),
Mo (max. 1,2%; bevorzugt max. 0,2%),
Ni (max. 0,4%; bevorzugt 0,15 bis 0,4%),
Al (0,01 bis 0,10%),
V (max. 0,15%),
Nb (max. 0,06%) und
Ti (max. 0,06%).

7. Verfahren nach einem der vorhergehenden Ansprüche, **dadurch gekennzeichnet, dass** das Rohrprodukt (1) eine Gefügestruktur aus gestrecktem, angelassenem Martensit aufweist, insbesondere mit einer durchschnittlichen martensitischen Paketgröße von d_{avg} < 3µm auf.

8. Verfahren nach einem der vorhergehenden Ansprüche, **dadurch gekennzeichnet, dass** das Rohrprodukt (1) eine Zugfestigkeit von mindestens 900 MPa und eine Übergangstemperatur von kleiner 233,15 Kelvin aufweist.

9. Verfahren nach einem der vorherigen Ansprüche, **dadurch gekennzeichnet, dass** ein Wirbelstromsensor (15) der Prüfsonde (9) derart positioniert wird, dass dieser nach dem Einfahren der Prüfsonde auf den Endbereich mit der Position des Übergangsbereichs (11-1, 11-2) korrespondiert.

10. Verfahren nach einem der vorherigen Ansprüche, **dadurch gekennzeichnet, dass**, das Prüfen mit unterschiedlichen Messfrequenzen oder Empfindlichkeiten der Wirbelstromsensoren (13, 14, 15) durchgeführt wird, um unterschiedliche Fehlerarten und/oder Fehlertiefen zu ermitteln.

11. Verfahren nach einem der vorherigen Ansprüche, **dadurch gekennzeichnet, dass** ein Prüfen auf Fehler im Übergangsbereich (11-1, 11-2) durch Verfahren eines Wirbelstromsensors (12,13, 14, 15) im einen Hinterschnitt des Übergangsbereichs (11-1, 11-2) der Innen- oder Außenwand (7, 8) erfolgt.

12. Prüfsonde (9), insbesondere zur Verwendung in einem Verfahren nach einem der vorhergehenden Ansprüche, **dadurch gekennzeichnet, dass** sie einen Steg (16) aufweist, der
a) wenigstens einen Arm (2, 3), an dessen Innenwand (17) wenigstens ein Wirbelstromsensor (13; 14, 15) angeordnet ist, und
b) ein Halteelement (4) zur Aufnahme wenigstens eines Wirbelstromsensors (12) miteinander verbindet,
wobei die Prüfsonde (9) als Rotationskörper um eine Mittellängsachse (19) ausgebildet ist, wobei der Steg (16) als Kreisscheibe, die beiden Arme (2, 3) zusammen als Hohlkörper mit kreisrunder Innenfläche und das Halteelement (4) als Körper mit kreisrunder Außenfläche ausgebildet sind.

13. Prüfsonde (9) nach Anspruch 12, **dadurch gekennzeichnet, dass** das Halteelement (4) äquidistant zu einem ersten und einem zweiten Arm (2, 3) auf dem Steg (16) angebracht ist.

## Claims

1. A method for manufacturing and testing of a pipe product (1) of steel comprising the following steps:
a) providing a steel pipe,
b) forming the steel pipe into the pipe product (1), wherein the steel pipe is formed at least in one of its end regions (10-1, 10-2),
c) testing the inner wall (7) and the outer wall (8) in at least one formed end region (10-1, 10-2) of the pipe product (1) for defects using at least one test probe (9) adapted to at least one formed end region (10-1, 10-2), which has at least one eddy current sensor (12) for testing the inner wall (7) and at least one eddy current sensor (13, 14, 15) for testing the outer wall (8) of the at least one formed end region (10-1, 10-2) of the pipe product, wherein several eddy current sensors (13, 14, 15) are provided on at least one arm (2, 3) and/or several sensors (12) are provided on an inner part (4).

2. The method according to claim 1, **characterized in that**, in step c), the inner wall and the outer wall are tested simultaneously.

3. The method according to claim 1 or 2, **characterized in that** the testing of the inner wall (7) and the outer wall (8) of the at least one formed end region (10-1, 10-2) of the pipe product (1) is carried out such that the pipe product (1) is fixed and the test probe (9) is moved axially in a rotating manner over the at least one formed end region (10-1, 10-2) of the pipe product, wherein the inner wall (7) and the outer wall (8) of the at least one formed end region (10-1, 10-2) of the pipe product are checked for defects by means of the eddy current sensors (12, 13, 14, 15).

4. The method according to any one of the preceding claims, **characterized in that** the testing of the inner wall (7) and the outer wall (8) in the at least one formed end region (10-1, 10-2) of the pipe product (1) is carried out following the final manufacturing steps, in particular the forming according to step b) and in particular in a sequential manner therewith.

5. The method according to any one of the preceding claims, **characterized in that** the testing is carried out following demagnetization of the at least one formed end region.

6. The method according to any one of the preceding claims, **characterized in that** the steel pipe provided consists of a steel alloy which, in addition to iron and melt-related impurities, contains the following alloying elements in mass percent:
C (0.07 to 0.50%; preferably 0.08 to 0.15%),
Si (0.01 to 0.60%; preferably 0.01 to 0.50%),
Mn (0.3 to 1.7%; preferably 1.0 to 1.7%),
Cr (max. 1.2%; preferably 0.2 to 0.9%),
Mo (max. 1.2%; preferably max. 0.2%),
Ni (max. 0.4%; preferably 0.15 to 0.4%),
Al (0.01 to 0.10%),
V (max. 0.15%),
Nb (max. 0.06%) and
Ti (max. 0.06%).

7. The method according to any one of the preceding claims, **characterized in that** the pipe product (1) has a microstructure of elongated, tempered martensite, in particular with an average martensitic packet size of d_{avg} < 3 µm.

8. The method according to any one of the preceding claims, **characterized in that** the pipe product (1) has a tensile strength of at least 900 MPa and a transition temperature of less than 233.15 Kelvin.

9. The method according to any one of the preceding claims, **characterized in that** an eddy current sensor (15) of the test probe (9) is positioned such that, after the test probe has been inserted into the end region, it corresponds to the position of the transition region (11-1, 11-2).

10. The method according to any one of the preceding claims, **characterized in that** the testing is carried out with different measuring frequencies or sensitivities of the eddy current sensors (13, 14, 15) in order to determine different types of defects and/or defect depths.

11. The method according to any one of the preceding claims, **characterized in that** testing for defects in the transition area (11-1, 11-2) is carried out by passing an eddy current sensor (12, 13, 14, 15) in an undercut of the transition area (11-1, 11-2) of the inner or outer wall (7, 8).

12. A test probe (9), in particular for use in a method according to any one of the preceding claims, **characterized in that** it has a web (16) which interconnects
a) at least one arm (2, 3) on the inner wall (17) of which at least one eddy current sensor (13; 14, 15) is arranged, and
b) a holding element (4) for receiving at least one eddy current sensor (12),
wherein the test probe (9) is configured as a rotary body about a central longitudinal axis (19), wherein the web (16) is configured as a circular disk, the two arms (2, 3) together being in the form of a hollow body with a circular inner face, and the holding element (4) is configured as a body with a circular outer face.

13. The test probe (9) according to claim 12, **characterized in that** the holding element (4) is mounted equidistantly to a first and a second arm (2, 3) on the web (16).

## Revendications

1. Procédé de fabrication et de vérification d'un produit tubulaire (1) en acier comprenant les étapes suivantes :
a) mise à disposition d'un tube en acier,
b) mise en forme du tube en acier en un produit tubulaire (1), dans lequel le tube en acier est mis en forme au moins dans une de ses zones d'extrémité (10-1, 10-2),
c) vérification de la paroi intérieure (7) et de la paroi extérieure (8) dans au moins une zone d'extrémité (10-1, 10-2) mise en forme du produit tubulaire (1) quant à des défauts au moyen d'au moins une sonde de vérification (9) adaptée à au moins une zone d'extrémité (10-1, 10-2) mise en forme, celle-ci présentant au moins un capteur à courants de Foucault (12) pour la vérification de la paroi intérieure (7) et au moins un capteur à courants de Foucault (13, 14, 15) pour la vérification de la paroi extérieure (8) de ladite au moins une zone d'extrémité (10-1, 10-2) mise en forme du produit tubulaire, dans lequel plusieurs capteurs à courants de Foucault (13, 14, 15) sont prévus sur au moins un bras (2, 3) et/ou plusieurs capteurs (12) étant prévus sur une partie intérieure (4).

2. Procédé selon la revendication 1, **caractérisé en ce qu'**à l'étape c), la paroi intérieure et la paroi extérieure sont vérifiées simultanément.

3. Procédé selon la revendication 1 ou la revendication 2, **caractérisé en ce que** la vérification de la paroi intérieure (7) et de la paroi extérieure (8) de ladite au moins une zone d'extrémité (10-1, 10-2) mise en forme du produit tubulaire (1) s'effectue de telle sorte que le produit tubulaire (1) soit fixé et que la sonde de vérification (9) soit déplacée axialement en rotation au-dessus de ladite au moins une zone d'extrémité (10-1, 10-2) mise en forme du produit brut, dans lequel, au moyen des capteurs à courants de Foucault (12, 13, 14, 15), à la fois la paroi intérieure (7) et la paroi extérieure (8) de ladite au moins une zone d'extrémité (10-1, 10-2) mise en forme du produit tubulaire sont vérifiées quant à des défauts.

4. Procédé selon l'une des revendications précédentes, **caractérisé en ce que** la vérification de la paroi intérieure (7) et de la paroi extérieure (8) dans ladite au moins une zone d'extrémité (10-1, 10-2) mise en forme du produit tubulaire (1) est effectuée à la suite des étapes finales de fabrication, en particulier à la suite de la mise en forme selon l'étape b), et en particulier enchaînée au cycle du procédé avec celle-ci.

5. Procédé selon l'une des revendications précédentes, **caractérisé en ce que** la vérification est effectuée à la suite d'une démagnétisation d'au moins une zone d'extrémité mise en forme.

6. Procédé selon l'une des revendications précédentes, **caractérisé en ce que** le tube en acier fourni est constitué d'un alliage d'acier qui, en dehors du fer et des impuretés dues à la fusion, présente les éléments d'alliage suivants en pourcentage massique :
C (0,07 à 0,50 % ; de préférence 0,08 à 0,15 %),
Si (0,01 à 0,60 % ; de préférence 0,01 à 0,50 %),
Mn (0,3 à 1,7 % ; de préférence 1,0 à 1,7 %),
Cr (au maximum 1,2 % ; de préférence 0,2 à 0,9 %),
Mo (au maximum 1,2 % ; de préférence au maximum 0,2 %),
Ni (au maximum 0,4 % ; de préférence 0,15 à 0,4 %),
Al (0,01 à 0,10 %),
V (au maximum 0,15 %),
Nb (au maximum 0,06 %), et
Ti (au maximum 0,06 %).

7. Procédé selon l'une des revendications précédentes, **caractérisé en ce que** le produit tubulaire (1) présente une microstructure constituée de martensite revenue allongée, en particulier avec une taille moyenne de paquet martensitique d_{avg} < 3 µm.

8. Procédé selon l'une des revendications précédentes, **caractérisé en ce que** le produit tubulaire (1) présente une résistance à la traction d'au moins 900 MPa et une température de transition inférieure à 233,15 kelvins.

9. Procédé selon l'une des revendications précédentes, **caractérisé en ce qu'**un capteur à courants de Foucault (15) de la sonde de vérification (9) est positionné de telle sorte qu'après l'introduction de la sonde de vérification dans la zone d'extrémité, celui-ci correspond à la position de la zone de transition (11-1, 11-2).

10. Procédé selon l'une des revendications précédentes, **caractérisé en ce que** la vérification est réalisée avec des fréquences de mesure ou des sensibilités différentes des capteurs à courants de Foucault (13, 14, 15) afin de déterminer différents types de défauts et/ou différentes profondeurs de défaut.

11. Procédé selon l'une des revendications précédentes, **caractérisé en ce qu'**une vérification quant à des défauts dans la zone de transition (11-1, 11-2) est réalisée par déplacement d'un capteur à courants de Foucault (12, 13, 14, 15) dans la contre-dépouille de la zone de transition (11-1, 11-2) de la paroi intérieure ou de la paroi extérieure (7, 8).

12. Sonde de vérification (9), en particulier destinée à être utilisée dans un procédé selon l'une des revendications précédentes, **caractérisée en ce qu'**elle présente une barrette (16) qui relie entre eux
a) au moins un bras (2, 3), sur la paroi intérieure (17) duquel au moins un capteur à courants de Foucault (13 ; 14, 15) est disposé, et
b) un élément de maintien (4) destiné à recevoir au moins un capteur à courants de Foucault (12),
dans laquelle la sonde de vérification (9) est formée sous la forme d'un corps de révolution autour d'un axe longitudinal médian (19), dans laquelle la barrette (16) est formée sous la forme d'un disque circulaire, les deux bras (2, 3) étant, ensemble, sous la forme d'un corps creux présentant une surface intérieure circulaire, et l'élément de maintien (4) étant sous la forme d'un corps présentant une surface extérieure circulaire.

13. Sonde de vérification (9) selon la revendication 12, **caractérisée en ce que** l'élément de maintien (4) est disposé à égale distance d'un premier et d'un second bras (2, 3) sur la barrette (16).
